# EUROPEAN PATENT APPLICATION

(11) **EP 3 263 039 A1**
(43) Date of publication of application: **03.01.2018**
(21) Application number: 15883353.3
(22) Date of filing: 26.11.2015
(51) Int. Cl.: A61B 8/12

(54) **ULTRASONIC PROBE**

(30) Priority: 25.02.2015 JP 2015035717
(71) Applicant: OLYMPUS CORPORATION, Hachioji-shi Tokyo 192-8507 (JP)
(72) Inventor: SATO, Sunao, Tokyo 192-8507 (JP)
(74) Representative: Schicker, Silvia
(86) International application number: PCT/JP2015/083252
(87) International publication number: WO 2016/136063

(57) **Abstract**

An ultrasound probe (100) includes: an ultrasound transmitting and receiving unit (101) configured to transmit ultrasound to a subject and receive an ultrasound echo reflected from the subject; an insulating portion (102) fixed on a distal end side of the ultrasound transmitting and receiving unit (101); and a balloon locking portion (103) having a groove portion (104) for locking an opening end part of a balloon that covers the ultrasound transmitting and receiving unit (101), the balloon locking portion (103) being fixed on a distal end side of the insulating portion (102) and formed of a material harder than the insulating portion (102).

## Description

### Field

The present invention relates to an ultrasound probe for observing an inside of a subject using ultrasound.

### Background

An endoscope is combined with an ultrasound probe to form an ultrasound endoscope. An example of the ultrasound probe for use in a subject includes a thin ultrasound probe that is used by passing through a treatment tool channel of an endoscope. Such an ultrasound probe is used in such a manner that an ultrasound transmitting and receiving unit is covered with a balloon, and the balloon is filled with water in order to achieve acoustic matching between the ultrasound transmitting and receiving unit and an observation target (such as an organ) (for example, refer to Patent Literature 1). The balloon is structured in such a manner that O rings are provided on opening portions at both ends of a cylindrical flexible membrane (rubber membrane). The balloon is attached when the O rings are fitted into grooves provided on a distal end side and a proximal end side of the ultrasound transmitting and receiving unit. Conventionally, such a balloon locking portion is formed of an insulation material such as resin in order to ensure electrical insulation properties between the balloon locking portion and the ultrasound transmitting and receiving unit.

### Citation List

### Patent Literature

Patent Literature 1: JP 2006-280407 A

### Summary

### Technical Problem

O rings of a balloon are fitted into balloon grooves so as to come into close contact with the balloon grooves in order to prevent leakage of water with which the balloon is filled. Therefore, a mechanical load is applied to a balloon locking portion in order to remove the balloon after an ultrasound probe is used, and damage such as a fine scratch is sometimes generated in the balloon locking portion due to the mechanical load. When the damage is generated in the balloon locking portion, water tightness between the balloon and the balloon groove cannot be ensured, and an examination with the use of the ultrasound probe might not be appropriately executed.

The present invention has been made in view of the foregoing, and an object of the invention is to provide an ultrasound probe that ensures insulation properties between a balloon locking portion and an ultrasound transmitting and receiving unit and does not allow the balloon locking portion to be damaged even when a mechanical load is applied to the balloon locking portion. Solution to Problem

In order to solve the above described problem and achieve the object, an ultrasound probe according to the invention includes: an ultrasound transmitting and receiving unit provided on an insertion portion configured to be inserted into a subject, the ultrasound transmitting and receiving unit being configured to transmit ultrasound to the subject and receive an ultrasound echo reflected from the subject; an insulating portion fixed on a distal end side of the ultrasound transmitting and receiving unit; and a balloon locking portion having a groove portion for locking an opening end part of a balloon that covers the ultrasound transmitting and receiving unit, the balloon locking portion being fixed on a distal end side of the insulating portion and formed of a material harder than the insulating portion.

In the ultrasound probe, the insulating portion has a side wall portion, the side wall portion projecting toward the ultrasound transmitting and receiving unit and being configured to be fitted into a distal end portion of the ultrasound transmitting and receiving unit.

In the ultrasound probe, the side wall portion circumferentially covers an outer periphery of the distal end portion of the ultrasound transmitting and receiving unit.

In the ultrasound probe, the side wall portion covers a plurality of positions on an outer periphery of the distal end portion of the ultrasound transmitting and receiving unit.

In the ultrasound probe, the balloon locking portion is formed of metal or carbon.

In the ultrasound probe, the balloon locking portion has a Vickers hardness of 50 or more.

In the ultrasound probe, the balloon locking portion and the insulating portion are integrally formed by means of two-color molding.

In the ultrasound probe, the balloon locking portion and the insulating portion are integrated by means of bonding, fitting, or screwing.

In the ultrasound probe, the balloon locking portion has, on an outer periphery thereof, a depression leading to the groove portion.

In the ultrasound probe, a depth of the depression becomes larger toward a bottom of the groove portion.

In the ultrasound probe, a protective member made of a material harder than the insulating portion is provided on a surface of the depression.

### Advantageous Effects of Invention

According to the present invention, since an insulating portion and a balloon locking portion formed of a material harder than the insulating portion are provided on a distal end side of an ultrasound transmitting and receiving unit, it is possible to ensure insulation properties between the balloon locking portion and the ultrasound transmitting and receiving unit, and to suppress damage to the balloon locking portion even when a mechanical load is applied to the balloon locking portion. Brief Description of Drawings

FIG. 1 is a schematic diagram illustrating an ultrasound endoscope system including an ultrasound probe according to a first embodiment of the present invention.
FIG. 2 is a schematic view illustrating an enlarged distal end hard portion of an ultrasound endoscope illustrated in FIG. 1.
FIG. 3 is a partial cross-sectional view of the distal end hard portion illustrated in FIG. 2.
FIG. 4 is a schematic view illustrating an ultrasound probe according to a second embodiment of the present invention.
FIG. 5 is a partial cross-sectional view of the ultrasound probe illustrated in FIG. 4.
FIG. 6 is a schematic view illustrating an ultrasound probe according to a third embodiment of the present invention.
FIG. 7 is a perspective view illustrating an ultrasound probe according to a fourth embodiment of the present invention.
FIG. 8 is a perspective view illustrating an ultrasound probe according to a fifth embodiment of the present invention.
FIG. 9 is a partial cross-sectional view of the ultrasound probe illustrated in FIG. 8.
FIG. 10 is a partial cross-sectional view illustrating an ultrasound probe according to a sixth embodiment of the present invention.
FIG. 11 is a partial cross-sectional view illustrating an ultrasound probe according to a first modification of the sixth embodiment of the present invention.
FIG. 12 is a partial cross-sectional view illustrating an ultrasound probe according to a second modification of the sixth embodiment of the present invention.
FIG. 13 is a partial cross-sectional view illustrating an ultrasound probe according to a third modification of the sixth embodiment of the present invention.
FIG. 14 is a partial cross-sectional view illustrating an ultrasound probe according to a fourth modification of the sixth embodiment of the present invention.

### Description of Embodiments

Hereinafter, ultrasound probes according to embodiments of the present invention will be described with reference to the drawings. The present invention is not limited by these embodiments. Although the following embodiments will be described using an ultrasound probe provided on an ultrasound endoscope as an example, the ultrasound probe according to the present invention can be generally applied to an ultrasound probe for use in a living body such as a thin ultrasound probe that is used in a subject by passing through a treatment tool channel of an endoscope.

The same reference signs are used to designate the same elements throughout the drawings. The drawings are only schematic, and a relation between thickness and width of each member and a ratio of each member or the like are different from actual ones. Dimensional relations and ratios between the elements in the different drawings may also be different from one another.

### (First Embodiment)

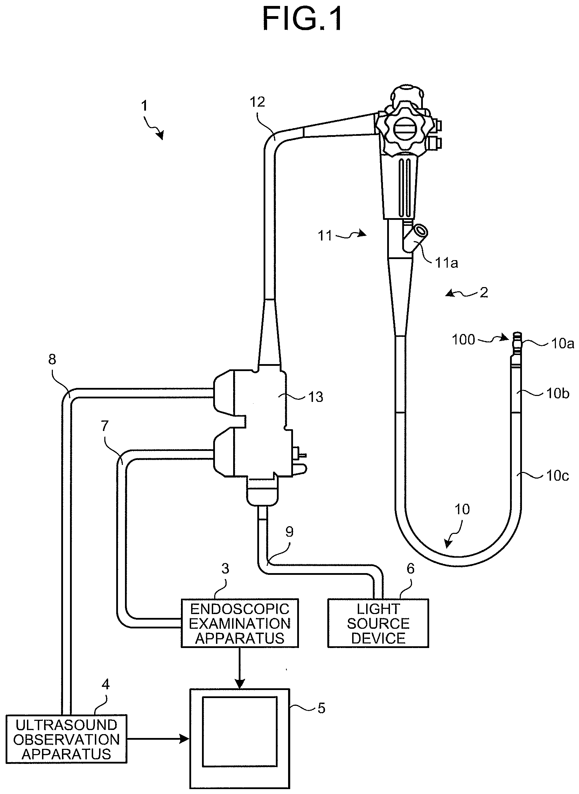

FIG. 1 is a schematic diagram illustrating an ultrasound endoscope system according to a first embodiment of the present invention. An ultrasound endoscope system 1 illustrated in FIG. 1 includes an ultrasound endoscope 2, an endoscopic examination apparatus 3, an ultrasound observation apparatus 4, a display device 5, a light source device 6, a video cable 7 that connects the ultrasound endoscope 2 to the endoscopic examination apparatus 3, an ultrasound cable 8 that connects the ultrasound endoscope 2 to the ultrasound observation apparatus 4, and a light source cable 9 that connects the ultrasound endoscope 2 to the light source device 6.

The ultrasound endoscope 2 is a combination of an ultrasound probe with an endoscopic examination unit having an image sensor and an observation optical system including a lens or the like. The ultrasound endoscope 2 has an endoscopic examination function and an ultrasound observation function. The endoscopic examination apparatus 3 controls the endoscopic examination function and processes an output signal output from the ultrasound endoscope 2 by means of the endoscopic examination. The ultrasound observation apparatus 4 controls the ultrasound observation function and processes an output signal output from the ultrasound endoscope 2 by means of the ultrasound observation. The display device 5 acquires signals output from, for example, the endoscopic examination apparatus 3 and the ultrasound observation apparatus 4, and appropriately displays at least one of an endoscope image and an ultrasound tomographic image. The light source device 6 includes a light source for supplying illumination light for performing the endoscopic examination.

The ultrasound endoscope 2 includes an insertion portion 10, an operating unit 11 continuously provided on a proximal end side of the insertion portion 10, and a universal cable 12 extending from a side portion of the operating unit 11. The insertion portion 10 is inserted into a body to transmit an ultrasound signal in a subject and receive the ultrasound signal reflected in the subject. The universal cable 12 has a connector unit 13 provided at an end portion different from an end portion close to the operating unit 11. The connector unit 13 is connected to each of the video cable 7, the ultrasound cable 8, and the light source cable 9.

The insertion portion 10 includes a distal end hard portion 10a formed of a hard member, a curve portion 10b configured to be freely curved, and a flexible pipe portion 10c having flexibility, which are continuously provided in order from a distal end side. A proximal end of the flexible pipe portion 10c is continuously provided on a distal end side of the operating unit 11. An ultrasound transmitting and receiving unit 101 to be described later is arranged on the distal end hard portion 10a.

A treatment tool insertion opening 11a for introducing a treatment tool such as a puncture needle into the body is provided in the operating unit 11. A treatment tool insertion passage is provided inside the insertion portion 10, and the treatment tool insertion opening 11a serves as an insertion opening for the treatment tool insertion passage.

The ultrasound endoscope 2 and the endoscopic examination apparatus 3 are electrically connected by the video cable 7 connected to the connector unit 13. The ultrasound endoscope 2 and the ultrasound observation apparatus 4 are electrically connected by the ultrasound cable 8 connected to the connector unit 13. The light source cable 9 is an optical fiber cable, and the ultrasound endoscope 2 and the light source device 6 guide the illumination light from the light source of the light source device 6 to the ultrasound endoscope 2 by means of the light source cable 9 connected to the connector unit 13.

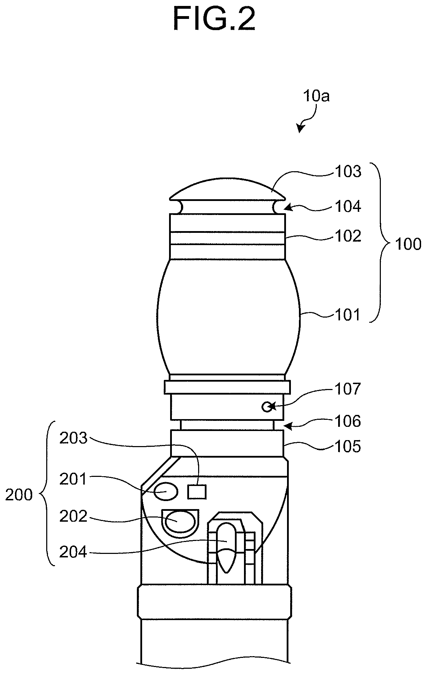

FIG. 2 is a schematic view illustrating the distal end hard portion of the ultrasound endoscope 2. The distal end hard portion 10a located on the distal end side of the insertion portion 10 includes an ultrasound probe 100 for observing an affected tissue (organ or the like) as an observation target, by means of ultrasound and an endoscopic examination unit 200 for directly and optically observing a surface of the organ or the like.

The ultrasound probe 100 includes the ultrasound transmitting and receiving unit 101, an insulating portion 102, a balloon locking portion 103, and a signal line (not illustrated). The ultrasound transmitting and receiving unit 101 transmits the ultrasound and receives the ultrasound (ultrasound echo) reflected from the observation target. The insulating portion 102 is provided on a distal end side of the ultrasound transmitting and receiving unit 101. The balloon locking portion 103 is provided on a distal end side of the insulating portion 102 and configured to lock a balloon that covers the ultrasound transmitting and receiving unit 101. The signal line transmits and receives an electric signal between the ultrasound transmitting and receiving unit 101 and the ultrasound observation apparatus 4. The balloon is structured in such a manner that O rings are provided on opening portions at both ends of a cylindrical flexible membrane (rubber membrane). One of the O rings is fit and fixed into a groove portion 104 formed on an outer periphery of the balloon locking portion 103. A detailed configuration of the ultrasound probe 100 will be described later.

The ultrasound transmitting and receiving unit 101 is joined to the endoscopic examination unit 200 via a joint portion 105. A groove portion 106 into which the other O ring provided on the balloon is fit is formed on the joint portion 105. A feed-water inlet 107 for supplying water with which the balloon is filled is provided in a region of the joint portion 105 which is closer to the ultrasound transmitting and receiving unit 101 than the groove portion 106.

The endoscopic examination unit 200 includes an illumination lens 201, an objective lens 202, an air/water supply port 203, and a suction/forceps port 204. The illumination lens 201 guides the illumination light to the observation target (surface of the organ or the like). The objective lens 202 collects the reflected light reflected from the surface of the organ or the like. The air/water supply port 203 is configured to supply gas or liquid into a body cavity. The suction/forceps port 204 is configured to suck gas or liquid in the body cavity, and a pair of forceps is configured to be attached to the suction/forceps port 204.

When the inside of the subject is observed using the above-mentioned ultrasound endoscope system 1, the O rings provided at both ends of the balloon are fitted into the groove portion 104 of the balloon locking portion 103 and the groove portion 106 of the joint portion 105, and the insertion portion 10 is inserted into the subject, with the O rings fixed. Then, water is taken through the feed-water inlet 107, and the balloon is filled with the water. In this state, the balloon is brought into contact with the observation target, and the ultrasound observation is performed. In addition, the observation target is illuminated via the illumination lens 201 to optically observe the inside of the subject.

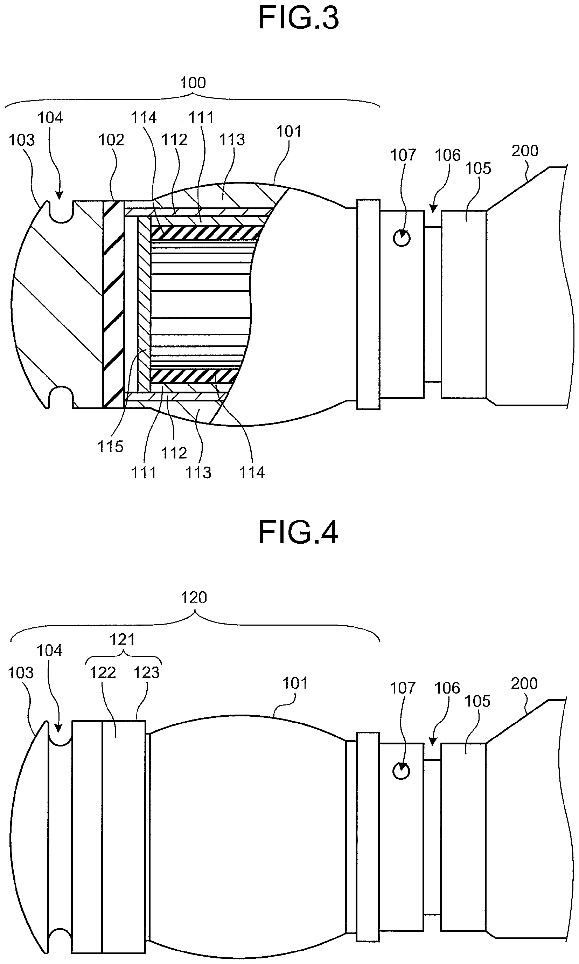

Next, the configuration of the ultrasound probe 100 will be described in detail. FIG. 3 is a partial cross-sectional view of the distal end hard portion illustrated in FIG. 2. The ultrasound transmitting and receiving unit 101 is what is called an electronic radial scanning probe in which a plurality of rectangular piezoelectric elements 111 is annularly arrayed. An acoustic matching layer 112 and an acoustic lens 113 are laminated on one end surface (ultrasound transmitting/receiving surface on an outer peripheral side of the ultrasound transmitting and receiving unit 101) of each piezoelectric element 111, and a backing material 114 is disposed on the other end side (surface on a central axis side of the ultrasound transmitting and receiving unit 101). The piezoelectric element 111, the acoustic matching layer 112, the acoustic lens 113, and the backing material 114 are supported by a support member 115 at an end portion. Illustrations of an electrode and a cable for applying a voltage to the piezoelectric element 111 are omitted from FIG. 3.

The insulating portion 102 is a disk-shaped member formed of a resin material such as polysulfone, polyetherimide, a polyphenylene oxide, and an epoxy resin. The insulating portion 102 is fixed to a distal end surface of the ultrasound transmitting and receiving unit 101 using an adhesive or the like.

The balloon locking portion 103 is formed in a columnar shape as a whole, and the groove portion 104 that locks an opening end part of the balloon is circumferentially formed on a side surface. A distal end surface of the balloon locking portion 103 may have a curved surface shape as illustrated in FIG. 2. Alternatively, the distal end surface may have a planar shape, and an outer periphery may be chamfered.

The balloon locking portion 103 is formed of a material harder than the insulating portion 102. Various indices can be used to represent the hardness of the material. As an example, the Vickers hardness is used to evaluate the hardness in the first embodiment.

For example, in a case where the insulating portion 102 is formed of the epoxy resin (Vickers hardness (HV) is 30 or less), the balloon locking portion 103 is formed of a material having a Vickers hardness of 50 or more and preferably 150 or more. Specific examples of such a material include stainless steel (Vickers hardness is about 150 to 300), a titanium alloy (Vickers hardness is about 110 to 150), a nickel alloy (Vickers hardness is about 150 to 450), and carbon (Vickers hardness is about a few thousand). As an example, the balloon locking portion 103 is formed of the stainless steel in the first embodiment.

The above-mentioned balloon locking portion 103 is fixed on the distal end side of the insulating portion 102. A method for fixing the balloon locking portion 103 to the insulating portion 102 is not particularly limited. For example, the balloon locking portion 103 may be fixed to the insulating portion 102 using an adhesive, or the balloon locking portion 103 and the insulating portion 102 may be integrally formed by means of two-color molding. Alternatively, a male screw portion may be formed on either the balloon locking portion 103 or the insulating portion 102, and a female screw portion may be formed on the other unit. Then, the male screw portion and the female screw portion may be screwed with each other. Still alternatively, a recessed portion may be provided in an end portion of either the balloon locking portion 103 or the insulating portion 102, and the other unit may be fitted into (pressed into) the recessed portion.

In the first embodiment, the two members, namely, the insulating portion 102 and the balloon locking portion 103 formed of the material harder than the insulating portion 102, are provided on the distal end side of the ultrasound transmitting and receiving unit 101 for the following reason. The inside of the ultrasound transmitting and receiving unit 101 is provided with an electrode, a signal line, and a ground wire or the like for applying a voltage to the piezoelectric element 111 that converts the electric signal and the ultrasound signal, and they are protectively grounded (refer to JP 2006-212077 A). Therefore, in order to insulate the ultrasound transmitting and receiving unit 101 from the water with which the balloon is filled when the ultrasound probe 100 is used, the insulating portion 102 is provided at the distal end.

With regard to the balloon locking portion 103, when the balloon attached to the groove portion 104 is removed after the ultrasound probe 100 is used, a mechanical load is sometimes applied to the balloon locking portion 103. In some cases, the balloon is picked out of the groove portion 104 using a thin instrument or the like. Therefore, in the first embodiment, the balloon locking portion 103 is formed of the material harder than the insulating portion 102 including the resin material. Consequently, even when the mechanical load is applied to the balloon locking portion 103, damage to the balloon locking portion 103 can be suppressed, and the life of the ultrasound probe 100 can be prolonged.

In the above-mentioned first embodiment, the balloon locking portion 103 is formed of the material harder than the insulating portion 102. However, in a case where the insulating portion 102 is formed of a sufficiently hard insulation material (for example, the Vickers hardness is 50 or more and preferably 150 or more) such as, for example, engineering plastic, the balloon locking portion 103 does not necessarily need to be formed of the material harder than the insulating portion 102. In other words, a material having a hardness equivalent to that of the insulating portion 102 may be used as the material for the balloon locking portion 103, or a material having a smaller hardness than the insulating portion 102 may be used as the material for the balloon locking portion 103. In any case, the balloon locking portion 103 only needs to be formed of a material having such a hardness as to withstand the mechanical load that is applied when the balloon is removed from the groove portion 104.

### (Second Embodiment)

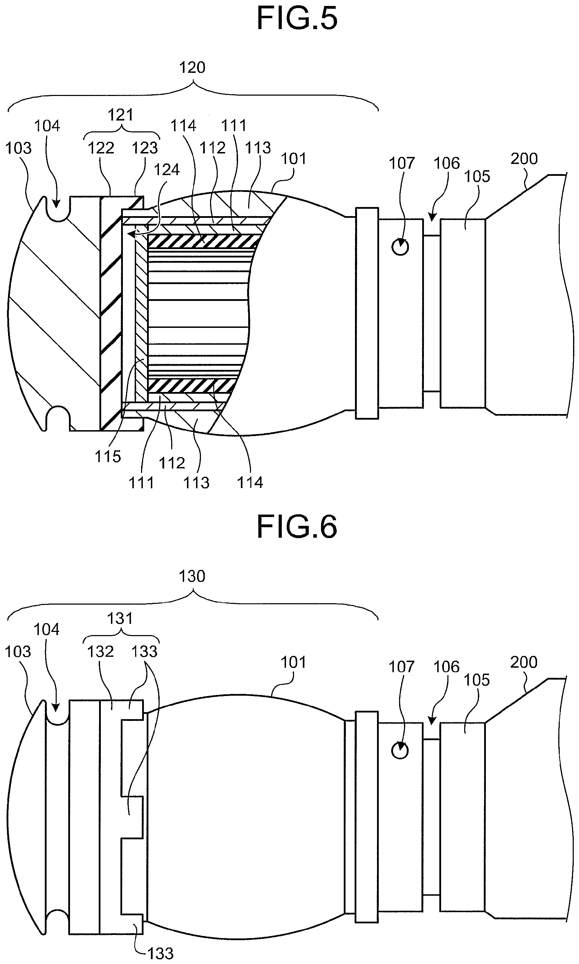

Next, a second embodiment of the present invention will be described. FIG. 4 is a schematic view illustrating an ultrasound probe according to the second embodiment of the present invention. FIG. 5 is a partial cross-sectional view of the ultrasound probe illustrated in FIG. 4.

As illustrated in FIGS. 4 and 5, an ultrasound probe 120 according to the second embodiment includes the ultrasound transmitting and receiving unit 101, an insulating portion 121, the balloon locking portion 103, and the signal line (not illustrated). The insulating portion 121 is provided on the distal end side of the ultrasound transmitting and receiving unit 101. The balloon locking portion 103 is provided on a distal end side of the insulating portion 121. The signal line transmits and receives the electric signal between the ultrasound transmitting and receiving unit 101 and the ultrasound observation apparatus 4. Among them, structures and functions of the ultrasound transmitting and receiving unit 101 and the balloon locking portion 103 are similar to those of the first embodiment.

The insulating portion 121 is formed in a cap shape having a bottom plate portion 122 and a side wall portion 123. The bottom plate portion 122 abuts on the end surface of the ultrasound transmitting and receiving unit 101. The side wall portion 123 is formed over the entire outer periphery of the bottom plate portion 122 and covers a side surface of the end portion of the ultrasound transmitting and receiving unit 101. An adhesive (not illustrated) is disposed in a recessed portion 124 in an inner periphery of the insulating portion 121, and the end portion of the ultrasound transmitting and receiving unit 101 is fitted into the recessed portion 124, whereby the insulating portion 121 is fixed to the ultrasound transmitting and receiving unit 101.

The insulating portion 121 is formed of a resin material such as polysulfone, polyetherimide, a polyphenylene oxide, and an epoxy resin. The insulating portion 121 is fixed to the balloon locking portion 103 by means of a method similar to that of the first embodiment. Specifically, both units may be bonded together using an adhesive, or both units may be integrally formed by means of the two-color molding. Alternatively, both units may be fixed by means of the screwing or the fitting (pressing).

As described above, the outer periphery of the end portion of the ultrasound transmitting and receiving unit 101 is structured to be circumferentially covered with the insulating portion 121 formed in the cap shape, whereby the bonded part of the insulating portion 121 and the ultrasound transmitting and receiving unit 101 can be protected from the water with which the periphery is filled when the ultrasound probe 120 is used. Consequently, the life of the ultrasound probe 120 can be prolonged. In addition, an electric discharge in a radial direction at the end portion of the ultrasound transmitting and receiving unit 101 can be suppressed.

### (Third Embodiment)

Next, a third embodiment of the present invention will be described. FIG. 6 is a schematic view illustrating an ultrasound probe according to the third embodiment of the present invention.

As illustrated in FIG. 6, an ultrasound probe 130 according to the third embodiment includes the ultrasound transmitting and receiving unit 101, an insulating portion 131, the balloon locking portion 103, and the signal line (not illustrated). The insulating portion 131 is provided on the distal end side of the ultrasound transmitting and receiving unit 101. The balloon locking portion 103 is provided on a distal end side of the insulating portion 131. The signal line transmits and receives the electric signal between the ultrasound transmitting and receiving unit 101 and the ultrasound observation apparatus 4. Among them, structures and functions of the ultrasound transmitting and receiving unit 101 and the balloon locking portion 103 are similar to those of the first embodiment.

The insulating portion 131 has a bottom plate portion 132 and a plurality of side wall portions 133. The bottom plate portion 132 abuts on the end surface of the ultrasound transmitting and receiving unit 101. The plurality of side wall portions 133 is formed at a plurality of positions on an outer periphery of the bottom plate portion 132, and abuts on the side surface of the end portion of the ultrasound transmitting and receiving unit 101. An adhesive (not illustrated) is disposed inside these side wall portions 133, and the end portion of the ultrasound transmitting and receiving unit 101 is fitted into the side wall portions 133, whereby the insulating portion 131 is fixed to the ultrasound transmitting and receiving unit 101.

The insulating portion 131 is formed of a resin material such as polysulfone, polyetherimide, a polyphenylene oxide, and an epoxy resin. The insulating portion 131 is fixed to the balloon locking portion 103 by means of a method similar to that of the first embodiment. Specifically, both units may be bonded together using an adhesive, or both units may be integrally formed by means of the two-color molding. Alternatively, both units may be fixed by means of the screwing or the fitting (pressing).

In the third embodiment, since the plurality of separated side wall portions 133 is provided, the attachment to the ultrasound transmitting and receiving unit 101 can be easily performed as compared with a case where the side wall portion 123 is integrally provided as illustrated in FIG. 4.

### (Fourth Embodiment)

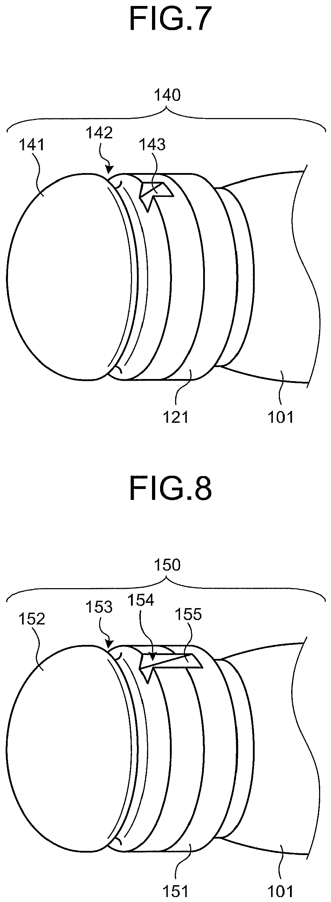

Next, a fourth embodiment of the present invention will be described. FIG. 7 is a perspective view illustrating an ultrasound probe according to the fourth embodiment of the present invention.

As illustrated in FIG. 7, an ultrasound probe 140 according to the fourth embodiment includes the ultrasound transmitting and receiving unit 101, the insulating portion 121, a balloon locking portion 141, and the signal line (not illustrated). The insulating portion 121 is provided on the distal end side of the ultrasound transmitting and receiving unit 101. The balloon locking portion 141 is provided on the distal end side of the insulating portion 121. The signal line transmits and receives the electric signal between the ultrasound transmitting and receiving unit 101 and the ultrasound observation apparatus 4 (refer to FIG. 1). Among them, a structure and a function of the ultrasound transmitting and receiving unit 101 are similar to those of the first embodiment. A structure and a function of the insulating portion 121 are similar to those of the second embodiment. In place of the insulating portion 121, the insulating portion 102 illustrated in FIG. 3 may be disposed, or the insulating portion 131 illustrated in FIG. 6 may be disposed.

The balloon locking portion 141 is formed in a columnar shape as a whole, and a groove portion 142 that locks the opening end part of the balloon is circumferentially formed on a side surface. A distal end surface of the balloon locking portion 141 may have a curved surface shape as illustrated in FIG. 7. Alternatively, the distal end surface may have a planar shape, and an outer periphery may be chamfered. A depression 143 leading to the groove portion 142 is provided on an outer periphery of the balloon locking portion 141. The depression 143 is used when the balloon fitted into the groove portion 142 is removed after the ultrasound probe 140 is used. The depression 143 is preferably inclined so that the depth gradually becomes deeper toward the bottom of the groove portion 142.

The balloon locking portion 141 is formed of a material harder than the insulating portion 121. Examples of the material include metal such as stainless steel and carbon. A condition for the hardness of the balloon locking portion 141 and a specific material are similar to those of the first embodiment. The above-mentioned balloon locking portion 141 is fixed to the insulating portion 121 by means of the method such as the bonding, the two-color molding, the screwing, and the fitting (pressing) in the same way as the first embodiment.

When the balloon is removed, an instrument such as a pair of tweezers is put into the depression 143, and the instrument only needs to be moved along the depression 143 so that the end portion of the balloon fitted into the groove portion 142 is scooped out. In this case, since the balloon locking portion 141 is formed of the hard material such as the stainless steel and the carbon as mentioned above, the damage to the balloon locking portion 141 can be suppressed even when the instrument comes into contact with the balloon locking portion 141.

### (Fifth Embodiment)

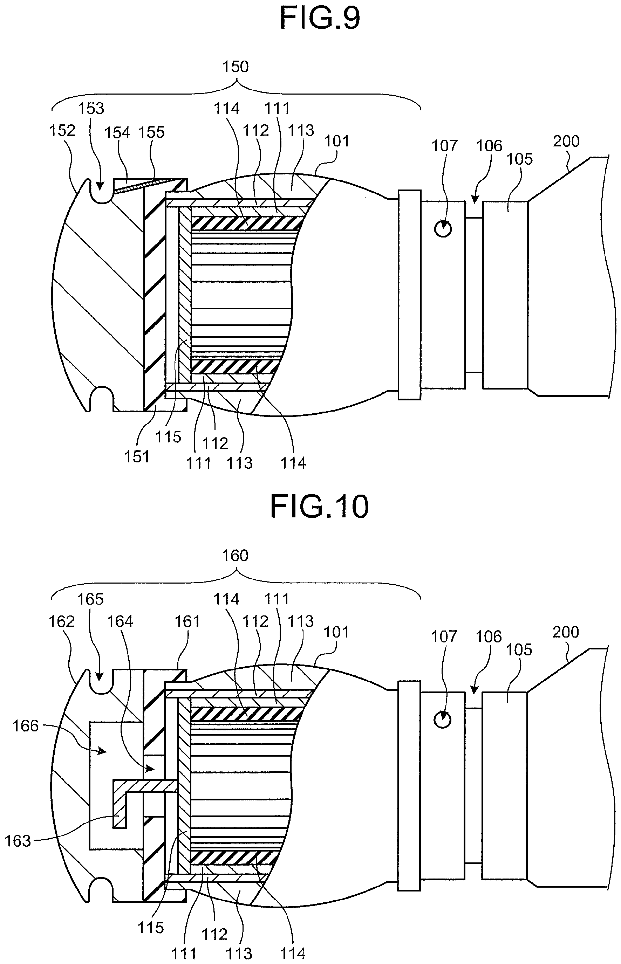

Next, a fifth embodiment of the present invention will be described. FIG. 8 is a perspective view illustrating an ultrasound probe according to the fifth embodiment of the present invention. FIG. 9 is a partial cross-sectional view of the ultrasound probe illustrated in FIG. 8.

As illustrated in FIGS. 8 and 9, the ultrasound probe 150 according to the fifth embodiment includes the ultrasound transmitting and receiving unit 101, an insulating portion 151, a balloon locking portion 152, and the signal line (not illustrated). The insulating portion 151 is provided on the distal end side of the ultrasound transmitting and receiving unit 101. The balloon locking portion 152 is provided on a distal end side of the insulating portion 151. The signal line transmits and receives the electric signal between the ultrasound transmitting and receiving unit 101 and the ultrasound observation apparatus 4 (refer to FIG. 1). Among them, a structure and a function of the ultrasound transmitting and receiving unit 101 are similar to those of the first embodiment.

The insulating portion 151 is formed in a cap shape in the same way as the second embodiment (refer to the insulating portion 121 in FIG. 5). Alternatively, the insulating portion 151 may be formed in a disk shape in the same way as the first embodiment (refer to the insulating portion 102 in FIG. 3), or may be shaped to have a plurality of side wall portions in the same way as the third embodiment (refer to the insulating portion 131 in FIG. 6). The insulating portion 151 is formed of a resin material such as polysulfone, polyetherimide, a polyphenylene oxide, and an epoxy resin.

The balloon locking portion 152 is formed in a columnar shape as a whole, and a groove portion 153 that locks the opening end part of the balloon is circumferentially formed on a side surface. A distal end surface of the balloon locking portion 152 may have a curved surface shape as illustrated in FIG. 9. Alternatively, the distal end surface may have a planar shape, and an outer periphery may be chamfered. The balloon locking portion 152 is formed of a material harder than the insulating portion 151. Examples of the material include metal such as stainless steel and carbon. A condition for the hardness of the balloon locking portion 152 and a specific material are similar to those of the first embodiment. The above-mentioned balloon locking portion 152 is fixed to the insulating portion 151 by means of the method such as the bonding, the two-color molding, the screwing, and the fitting (pressing) in the same way as the first embodiment.

A depression 154 leading to the groove portion 153 is provided on the insulating portion 151 and the balloon locking portion 152, and a protective member 155 is disposed on surfaces (a bottom surface and side surfaces) of the depression 154. The depression 154 is used when the balloon fitted into the groove portion 153 is removed after the ultrasound probe 150 is used. The depression 154 is preferably inclined so that the depth gradually becomes deeper toward the bottom of the groove portion 153.

The protective member 155 is formed of a material harder than the insulating portion 151. For example, in a case where the insulating portion 151 is formed of the epoxy resin having a Vickers hardness of 30 or less, it is preferable that the protective member 155 is formed of a material having a Vickers hardness of 50 or more and preferably 150 or more. More specifically, examples of the material for the protective member 155 include metal such as stainless steel, a titanium alloy, and a nickel alloy and carbon.

A method for disposing the protective member 155 in the depression 154 is not particularly limited. As an example, a plate-shaped or membranous member formed of metal or carbon only needs to be bonded to the depression 154.

As described above, the depression 154 is provided to extend over the insulating portion 151 and the balloon locking portion 152, whereby the work of removing the balloon from the groove portion 153 can be easily performed. Since the insulating portion 151 is covered with the protective member 155, the damage to the insulating portion 151 can be suppressed even when the instrument (such as a pair of tweezers) used for the removal of the balloon comes into contact with the depression 154.

### (Sixth Embodiment)

Next, a sixth embodiment of the present invention will be described. FIG. 10 is a partial cross-sectional view illustrating an ultrasound probe according to the sixth embodiment of the present invention.

As illustrated in FIG. 10, an ultrasound probe 160 according to the sixth embodiment includes the ultrasound transmitting and receiving unit 101, an insulating portion 161, a balloon locking portion 162, and the signal line (not illustrated). The insulating portion 161 is provided on the distal end side of the ultrasound transmitting and receiving unit 101. The balloon locking portion 162 is provided on a distal end side of the insulating portion 161. The signal line transmits and receives the electric signal between the ultrasound transmitting and receiving unit 101 and the ultrasound observation apparatus 4 (refer to FIG. 1). Among them, although a structure and a function of the ultrasound transmitting and receiving unit 101 are similar to those of the first embodiment, a fall prevention member 163 projecting toward a distal end side is provided on the support member 115 in the sixth embodiment.

The outer shape of the insulating portion 161 is a cap shape in the same way as the second embodiment (refer to the insulating portion 121 in FIG. 5). Alternatively, the outer shape of the insulating portion 161 may be a disk shape in the same way as the first embodiment (refer to the insulating portion 102 in FIG. 3), or may be a shape having a plurality of side wall portions in the same way as the third embodiment (refer to the insulating portion 131 in FIG. 6). A through hole 164 that allows the fall prevention member 163 to pass therethrough is formed in the insulating portion 161. The above-mentioned insulating portion 161 is formed of a resin material such as polysulfone, polyetherimide, a polyphenylene oxide, and an epoxy resin.

The balloon locking portion 162 is formed in a columnar shape as a whole, and a groove portion 165 that locks the opening end part of the balloon is circumferentially formed on a side surface. An opening 166 communicating with the through hole 164 of the insulating portion 161 is provided inside the balloon locking portion 162. The above-mentioned balloon locking portion 162 is formed of a material harder than the insulating portion 161. Examples of the material include metal such as stainless steel and carbon. A condition for the hardness of the balloon locking portion 162 and a specific material are similar to those of the first embodiment. The balloon locking portion 162 is fixed to the insulating portion 161 by means of the method such as the bonding, the two-color molding, the screwing, and the fitting (pressing) in the same way as the first embodiment.

The fall prevention member 163 is such a member that a distal end of a rod-shaped member is bent into a hook. The length of the bent distal end part is preferably equal to or longer than the inner diameter of the through hole 164 of the insulating portion 161. The above-mentioned fall prevention member 163 is preferably formed of an insulation material such as hard plastic and carbon. Alternatively, in a case where the fall prevention member 163 is formed of a conductive material such as metal, it is preferable that a surface of the fall prevention member 163 is coated with an insulation material such as resin. Consequently, a short circuit between the fall prevention member 163 and the balloon locking portion 162 can be prevented.

When the ultrasound probe 160 is assembled, the fall prevention member 163 is caused to pass through the through hole 164 of the insulating portion 161, and the part bent into the hook is caused to project toward the opening 166 of the balloon locking portion 162. In this state, the insulating portion 161 and the ultrasound transmitting and receiving unit 101 are bonded together.

Since the above-mentioned fall prevention member 163 is provided, the insulating portion 161 is caught on the fall prevention member 163 even if the insulating portion 161 comes off the ultrasound transmitting and receiving unit 101. Therefore, the insulating portion 161 and the balloon locking portion 162 can be prevented from falling off.

### (First Modification)

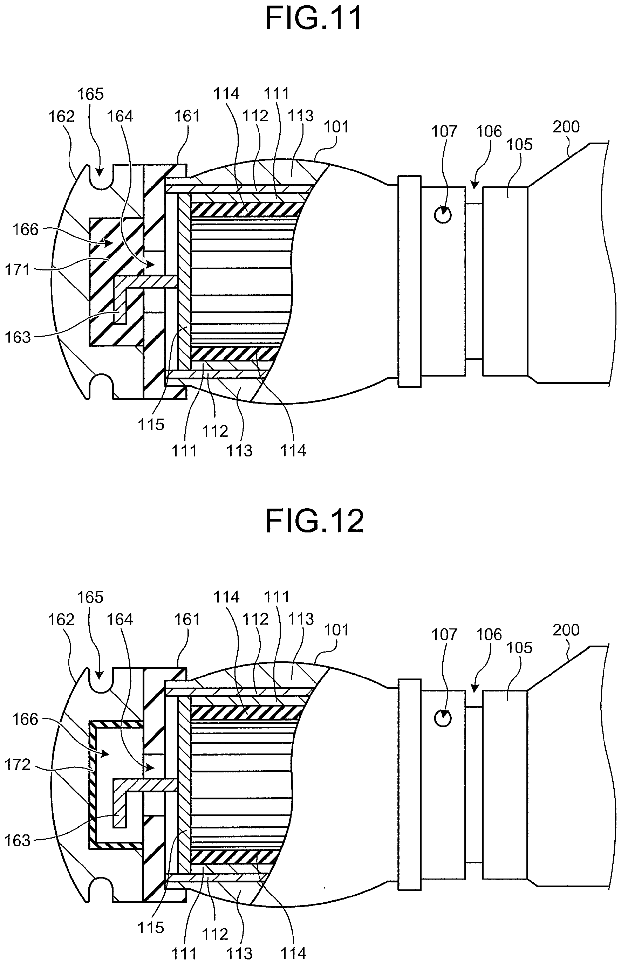

Next, a first modification of the sixth embodiment of the present invention will be described. FIG. 11 is a partial cross-sectional view illustrating an ultrasound probe according to the first modification of the sixth embodiment of the present invention.

As illustrated in FIG. 11, in the first modification, the opening 166 of the balloon locking portion 162 is filled with an adhesive 171 having insulation properties. Alternatively, the through hole 164 of the insulating portion 161 may also be filled with the adhesive 171.

Consequently, even when the fall prevention member 163 is formed of the conductive material such as the metal, the short circuit between the fall prevention member 163 and the balloon locking portion 162 can be prevented, and the ultrasound transmitting and receiving unit 101, the insulating portion 161, and the balloon locking portion 162 can be fixed more firmly.

### (Second Modification)

Next, a second modification of the sixth embodiment of the present invention will be described. FIG. 12 is a partial cross-sectional view illustrating an ultrasound probe according to the second modification of the sixth embodiment of the present invention.

As illustrated in FIG. 12, in the second modification, an insulation coat 172 is formed on an inner wall surface of the opening 166 of the balloon locking portion 162. The insulation coat 172 is formed in such a manner that, for example, an adhesive having insulation properties or a liquid insulation coating agent is applied to the inner wall surface of the opening 166 and hardened.

Since the above-mentioned insulation coat 172 is provided, even when the fall prevention member 163 is formed of the conductive material such as the metal, the short circuit between the fall prevention member 163 and the balloon locking portion 162 can be prevented.

### (Third Modification)

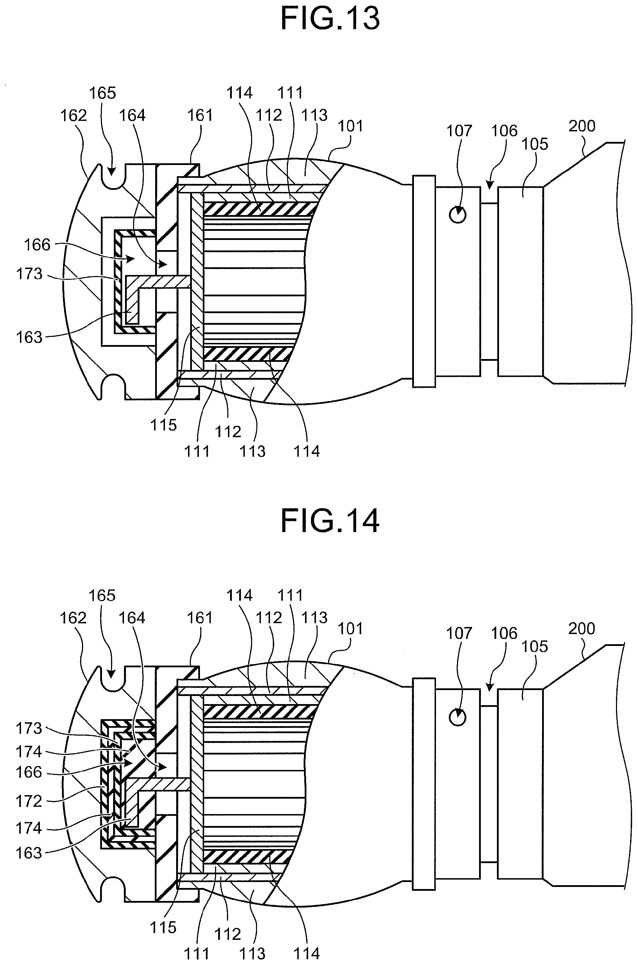

Next, a third modification of the sixth embodiment of the present invention will be described. FIG. 13 is a partial cross-sectional view illustrating an ultrasound probe according to the third modification of the sixth embodiment of the present invention.

As illustrated in FIG. 13, in the third modification, an insulation member 173 that covers the through hole 164 is provided on a surface of the insulating portion 161 close to the balloon locking portion 162. The insulation member 173 is formed of an insulation material such as resin, and has a sheet shape or a plate shape.

The short circuit between the fall prevention member 163 and the balloon locking portion 162 can also be prevented when the above-mentioned insulation member 173 is provided.

### (Fourth Modification)

Next, a fourth modification of the sixth embodiment of the present invention will be described. FIG. 14 is a partial cross-sectional view illustrating an ultrasound probe according to the fourth modification of the sixth embodiment of the present invention.

As illustrated in FIG. 14, in the fourth modification, the insulation coat 172 is formed on the inner wall surface of the opening 166 of the balloon locking portion 162, and the insulation member 173 is provided on the insulating portion 161. Furthermore, a space between the insulation coat 172 and the insulation member 173 and a space inside the insulation member 173 are filled with an adhesive 174 having insulation properties. Alternatively, the through hole 164 of the insulating portion 161 may also be filled with the adhesive 174.

Consequently, even when the fall prevention member 163 is formed of the conductive material such as the metal, the short circuit between the fall prevention member 163 and the balloon locking portion 162 can be prevented more reliably, and the ultrasound transmitting and receiving unit 101, the insulating portion 161, and the balloon locking portion 162 can be fixed more firmly.

In the first to sixth embodiments and the first to fourth modifications of the present invention described above, the type of the ultrasound transmitting and receiving unit 101 is the electronic radial scanning type in which the plurality of rectangular piezoelectric elements 111 is annularly arrayed. However, the first to sixth embodiments and the first to fourth modifications may also be applied to an ultrasound probe having a linear ultrasound transmitting and receiving unit in which a plurality of piezoelectric elements is linearly arrayed, a convex ultrasound transmitting and receiving unit in which a plurality of piezoelectric elements is arrayed in a protruding curved surface shape, or a mechanical radial scanning ultrasound transmitting and receiving unit in which a single piezoelectric element is mechanically rotated.

The above-described present invention is not limited to the first to sixth embodiments and the first to fourth modifications thereof, and can be variously changed in accordance with the specification or the like. For example, some components may be excluded from all the components described in the first to sixth embodiments and the first to fourth modifications thereof to form the present invention. It is obvious from the above description that various other embodiments can be provided within the range of the present invention.

### Industrial Applicability

As described above, the ultrasound probe according to the present invention is useful in ensuring insulation properties between a balloon locking portion and an ultrasound transmitting and receiving unit and suppressing damage to the balloon locking portion even when a mechanical load is applied to the balloon locking portion.

### Reference Signs List

- 1: ULTRASOUND ENDOSCOPE SYSTEM
- 2: ULTRASOUND ENDOSCOPE
- 3: ENDOSCOPIC EXAMINATION APPARATUS
- 4: ULTRASOUND OBSERVATION APPARATUS
- 5: DISPLAY DEVICE
- 6: LIGHT SOURCE DEVICE
- 7: VIDEO CABLE
- 8: ULTRASOUND CABLE
- 9: LIGHT SOURCE CABLE
- 10: INSERTION PORTION
- 11: OPERATING UNIT
- 12: UNIVERSAL CABLE
- 13: CONNECTOR UNIT
- 100, 120, 130, 140, 150, 160: ULTRASOUND PROBE
- 101: ULTRASOUND TRANSMITTING AND RECEIVING UNIT
- 102, 121, 131, 151, 161: INSULATING PORTION
- 103, 141, 152, 162: BALLOON LOCKING PORTION
- 104, 106, 142, 153, 165: GROOVE PORTION
- 105: JOINT PORTION
- 107: FEED-WATER INLET
- 111: PIEZOELECTRIC ELEMENT
- 112: ACOUSTIC MATCHING LAYER
- 113: ACOUSTIC LENS
- 114: BACKING MATERIAL
- 115: SUPPORT MEMBER
- 122, 132: BOTTOM PLATE PORTION
- 123, 133: SIDE WALL PORTION
- 124: RECESSED PORTION
- 143, 154: DEPRESSION
- 155: PROTECTIVE MEMBER
- 163: FALL PREVENTION MEMBER
- 164: THROUGH HOLE
- 166: OPENING
- 171, 174: ADHESIVE
- 172: INSULATION COAT
- 173: INSULATION MEMBER
- 200: ENDOSCOPIC EXAMINATION UNIT
- 201: ILLUMINATION LENS
- 202: OBJECTIVE LENS
- 203: AIR/WATER SUPPLY PORT
- 204: SUCTION/FORCEPS PORT

## Claims

1. An ultrasound probe comprising:
an ultrasound transmitting and receiving unit provided on an insertion portion configured to be inserted into a subject, the ultrasound transmitting and receiving unit being configured to transmit ultrasound to the subject and receive an ultrasound echo reflected from the subject;
an insulating portion fixed on a distal end side of the ultrasound transmitting and receiving unit; and
a balloon locking portion having a groove portion for locking an opening end part of a balloon that covers the ultrasound transmitting and receiving unit, the balloon locking portion being fixed on a distal end side of the insulating portion and formed of a material harder than the insulating portion.

2. The ultrasound probe according to claim 1, wherein
the insulating portion has a side wall portion, the side wall portion projecting toward the ultrasound transmitting and receiving unit and being configured to be fitted into a distal end portion of the ultrasound transmitting and receiving unit.

3. The ultrasound probe according to claim 2, wherein
the side wall portion circumferentially covers an outer periphery of the distal end portion of the ultrasound transmitting and receiving unit.

4. The ultrasound probe according to claim 2, wherein
the side wall portion covers a plurality of positions on an outer periphery of the distal end portion of the ultrasound transmitting and receiving unit.

5. The ultrasound probe according to claim 1, wherein
the balloon locking portion is formed of metal or carbon.

6. The ultrasound probe according to claim 1, wherein
the balloon locking portion has a Vickers hardness of 50 or more.

7. The ultrasound probe according to claim 1, wherein
the balloon locking portion and the insulating portion are integrally formed by means of two-color molding.

8. The ultrasound probe according to claim 1, wherein
the balloon locking portion and the insulating portion are integrated by means of bonding, fitting, or screwing.

9. The ultrasound probe according to claim 1, wherein
the balloon locking portion has, on an outer periphery thereof, a depression leading to the groove portion.

10. The ultrasound probe according to claim 9, wherein
a depth of the depression becomes larger toward a bottom of the groove portion.

11. The ultrasound probe according to claim 9 or 10, wherein
a protective member made of a material harder than the insulating portion is provided on a surface of the depression.
